# EUROPÄISCHE PATENTSCHRIFT

(11) **EP 2 932 987 B1**
(45) Veröffentlichungstag und Bekanntmachung des Hinweises auf die Patenterteilung: **15.11.2017**
(21) Anmeldenummer: 15160717.3
(22) Anmeldetag: 25.03.2015
(51) Int. Cl.: A61L 24/06, A61L 24/04, A61L 27/16

(54) **POLYMETHYLMETHACRYLAT-KNOCHENZEMENT**
POLYMETHYL METHACRYLATE BONE CEMENT
CIMENT OSSEUX EN POLYMÉTHACRYLATE DE MÉTHYLE

(30) Priorität: 14.04.2014 DE 102014105267
(43) Veröffentlichungstag der Anmeldung: 21.10.2015
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A1- 2 664 349
- EP-A2- 2 497 504
- EP-A2- 2 550 979
- EP-A2- 2 687 239
- US-A- 5 902 839

## Beschreibung

Die Erfindung betrifft einen zweikomponentigen Polymethylmethacrylat-Knochenzement.

In der Medizin werden seit Jahrzehnten Polymethylmethacrylat-Knochenzemente zur dauerhaften mechanischen Fixierung von Totalgelenkendoprothesen eingesetzt. Diese basieren auf Pulver-Flüssigkeits-Systemen, wobei Methylmethacrylat üblicherweise als Monomer verwendet wird. Ein allgemeiner Überblick findet sich z.B. in K.-D. Kühn, Knochenzemente für die Endoprothetik: ein aktueller Vergleich der physikalischen und chemischen Eigenschaften handelsüblicher PMMA-Zemente, Springer-Verlag Berlin Heidelberg New York, 2001.

US 8536243 B2 beschreibt ein Pulver-Gel-Knochenzement-System. Bei diesem handelt es sich um eine Modifikation der konventionellen Pulver-Flüssigkeits-Knochenzemente. Die pulverförmige Komponente besteht aus einem Polymer, einen Initiator wie Dibenzoylperoxid und gegebenenfalls einem Röntgenopaker und Hydroxyapatit. Die gelartige Komponente umfasst ein Acrylmonomer wie Methylmethacrylat, einen Radikalinhibitor wie Hydrochinon, einen Aktivator wie N,N-Dimethyl-p-toluidin und ein im Acrylmonomer gelöstes Polymer mit einem mittleren Molekulargewicht von größer 1,000,000 g/mol.

Es wurden in jüngster Zeit auch Polymethylmethacrylat-Knochenzemente vorgeschlagen, die auf der Verwendung von Zementpasten beruhen. DE 102007052116 A1 betrifft einen einkomponentigen Knochenzement. DE 102007050762 B3 und DE 102008030312 A1 beschreiben zweikomponentige Knochenzemente aus zwei Zementpasten, die separat in geeigneten Kartuschen gelagert werden. Die Knochenzemente enthalten jeweils mindestens ein Monomer, geeignete polymere Bestandteile und ein Redoxinitiatorsystem. In den Knochenzementen wird meist Methylmethacrylat als Monomer verwendet.

EP 2 497 50 4 A2 beschreibt einen binären Pasten-Pulver-Knochenzement, wobei eine Pastenkomponente, die durch Lösen einer Pulverzusammensetzung in einer flüssigen Komponente hergestellt wird, und eine Pulverkomponente, die eine zweite Pulverzusammensetzung enthält, vermischt werden, ohne dass eine Ruhezeit notwendig ist.

In EP 2 664 349 A1 ist ein pastenförmiger Knochenzement offenbart, welcher aus zwei Pasten A und B hergestellt wird. Paste A enthält als Polymersationsbeschleuniger wenigstens ein Peroxid und als Co-Polymerisationsbeschleuniger wenigstens ein tertiäres Amin, während Paste B als Polymerisationsbeschleuniger eine Schwermetallverbindung und als Co-Polymerisationsbeschleuniger wenigstens ein Sulfimid enthält.

EP 2 687 239 A2 beschreibt einen pastenförmigen Knochenzement, der einen anorganischen Füllstoff mit einer BET-Oberfläche von mindestens 40 m²/g enthält. Der Knochenzement wird aus zwei Pasten hergestellt, von denen eine einen Polymerisationsinitiator und die andere einen Polymerisationsbeschleuniger enthält.

EP 2 550 979 A2 beschreibt einen Knochenzement, der aus zwei Pasten hergestellt wird, von denen eine einen radikalischen Polymerisationsinitiator und die andere einen Akzelerator, bei dem es sich um ein tertiäres Amin, Saccharin oder ein Schwermetallsalz handeln kann, enthält.

US 5,902,839 A offenbart einen Knochenzement geringer Porösität, der durch Mischen von mindestens zwei flüssigen Komponenten hergestellt wird. Eine dieser flüssigen Komponenten enthält einen Aktivator und einen Polymerisationsinhibitor, während die andere flüssige Komponente einen Initiator enthält.

Die eingesetzten Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Komponenten des Redoxinitiatorsystems zusammenwirken. Deshalb werden die Komponenten des Redoxinitiatorsystems in den separaten Zementpasten so angeordnet, dass sie keine radikalische Polymerisation auslösen können.

Die Zementpasten sind lagerstabil. Erst bei Vermischung der beiden Zementpasten zu einem Zementteig reagieren die zuvor getrennt in beiden Pasten gelagerten Komponenten des Redoxinitiatorsystems, wobei Radikale gebildet werden, welche die radikalische Polymerisation des enthaltenen Monomers auslösen. Die radikalische Polymerisation führt dann unter Verbrauch des Monomers zu Bildung von Polymeren, wobei der Zementteig aushärtet.

Zur Vermischung der Zementpasten werden üblicherweise statische Mischer eingesetzt, die dazu an 2-Komponentenkartuschen angebracht werden. Beim Auspressen der beiden Zementpasten aus den Kartuschen werden die beiden Zementpasten durch einen statischen Mischer gedrückt. Der Auspress- und der Vermischungsvorgang erfolgen dadurch gleichzeitig. Zur Vermischung der Zementpasten im statischen Mischer ist eine hohe Auspresskraft notwendig, weil der Druckabfall innerhalb des statischen Mischers an den Mischelementen sehr hoch ist. Dadurch ist es notwendig, leistungsfähige pneumatische oder mechanische Auspressvorrichtungen einzusetzen, um einen Austrag und eine Vermischung der Zementpasten zu erreichen. Diese pneumatischen oder auch mechanischen Auspressvorrichtungen sind technisch aufwendig und teuer.

Kostengünstig sind dagegen die bisher bei den auf Pulver-Flüssigkeits-Systemen beruhenden Polymethylmethacrylat-Knochenzementen üblichen manuell betriebenen Auspresspistolen, welche sich für diese Zemente eignen, jedoch für die Auspressung und Vermischung von Knochenzementpasten unter Verwendung von statischen Mischern nicht ausreichend leistungsfähig sind.

Bei konventionellen 2-Komponentenkartuschen ist das Volumenverhältnis der Paste A zur Paste B häufig 1:1, 1:2 oder 1:10. Je ungleicher die Volumina der mit statischen Mischern zu vermischenden Pasten sind, umso schwieriger ist es, eine homogen gemischte Paste zu erzielen. Deshalb sind bei größeren Volumenverhältnissen sehr viele Mischwendel notwendig. Je größer die Anzahl der benötigten Mischwendel ist, umso größer ist auch der Druckabfall beim Mischprozess im statischen Mischer. Das bedeutet, die Pasten müssen mit sehr großer Kraft durch den statischen Mischer gepresst werden. Bei manuell betriebenen Auspressvorrichtungen ist naturgemäß die maximal mögliche Auspresskraft beschränkt.

Die Knochenzemente enthalten in der Regel Röntgenopaker, damit der im Körper verwendete Zement im Röntgenbild sichtbar ist. Übliche Röntgenopaker, wie Zirkoniumdioxid und Bariumsulfat, besitzen aber eine hohe Dichte. Wenn sie in Pasten enthalten sind, besteht daher die Gefahr, dass die Röntgenopaker sedimentieren, was zu einer Beeinträchtigung der Qualität des Zements führen kann.

Aufgabe der Erfindung war die Bereitstellung eines zweikomponentigen Knochenzements, der die vorstehend beschriebenen Nachteile des Standes der Technik überwindet. Insbesondere sollen die beiden Komponenten manuell ohne Verwendung von statischen Mischern vermischt werden können, um einen sofort klebfreien, plastisch verformbaren Zementteig erhalten zu können. Weiterhin soll der zu entwickelnde Polymethylmethacrylat-Knochenzement so beschaffen sein, dass eine Sedimentation des Röntgenopakers vermieden wird. Eine Wechselwirkung des Monomers mit gegebenenfalls zugesetzten pharmazeutischen Wirkstoffen sollte ebenfalls vermieden werden. Desweiteren soll die Herstellung der Komponenten möglichst einfach und damit preisgünstig sein.

Die Erfindung basiert auf der Idee, einen Polymethylmethacrylat-Knochenzement zu entwickeln, bei dem es möglich ist, eine pastenförmige Komponente mit einer pulverförmigen Komponente manuell so zu vermischen, dass ein selbst aushärtender Zementteig entsteht, der unter Verwendung von üblichen manuell zu betätigenden Zementiervorrichtungen ausgepresst und appliziert werden kann. Die Idee besteht darin, den Mischvorgang und den Auspressvorgang des Polymethylmethacrylat-Knochenzementes zeitlich nacheinander ablaufen zu lassen, im Gegensatz zu Zweikomponenten-Polymethylmethacrylat-Knochenzementen, die aus zwei Pasten aufgebaut sind, bei denen die Vermischung und Applikation zeitgleich während des Auspressens der beiden Pasten durch einen statischen Mischer erfolgt. Für die Vermischung von zwei Pasten ist ein relativ hoher Energieeintrag notwendig. Dadurch erfordert die Applikation von Zweikomponenten-Polymethylmethacrylat-Knochenzementen Auspressvorrichtungen mit eine hohen Auspresskraft.

Es wurde überraschend gefunden, dass es möglich ist, durch manuelles Vermischen einer pastenförmigen Komponente wie nachstehend definiert mit einer zweiten, pulverförmigen Komponente wie nachstehend definiert einen klebfreien, plastisch verformbaren Knochenzementteig herzustellen, der selbstständig durch radikalische Polymerisation aushärtet.

Die Aufgabe der Erfindung wurde daher mit einem zweikomponentigen Knochenzement gelöst, der
A) eine Paste als Komponente A umfassend
   a1) Methylmethacrylat,
   a2) mindestens ein in Methylmethacrylat lösliches Polymer mit einer zahlenmittleren Molmasse kleiner 500.000 Dalton,
   a3) mindestens ein in Methylmethacrylat unlösliches, partikuläres Polymer, das eine Partikelgröße D50 kleiner 50 µm hat,
   a4) mindestens ein in Methylmethacrylat lösliches Hydroperoxid und
   a5) mindestens ein in Methylmethacrylat lösliches tertiäres Amin und
B) ein Pulver als Komponente B umfassend
   b1) mindestens einen partikulären Röntgenopaker, der eine Partikelgröße D50 kleiner 50 µm hat,
   b2) mindestens ein in Methylmethacrylat lösliches Schwermetallsalz und
   b3) mindestens ein in Methylmethacrylat lösliches Reduktionsmittel.
umfasst.

Nach Vermischung der pastenförmigen Komponente A mit der pulverförmigen Komponente B entsteht ohne Einhaltung einer Wartezeit sofort ein klebfreier, plastisch verformbarer Zementteig, der selbstständig durch radikalische Polymerisation aushärtet. Bei dem zweikomponentigen Knochenzement handelt es sich um einen Polymethylmethacrylat-Knochenzement.

Ein wesentlicher Vorteil der Erfindung besteht darin, dass der erfindungsgemäße Zweikomponentenzement in einem deutlich preisgünstigeren Zementiersystem gelagert und aus diesem appliziert werden kann im Vergleich zu den bekannten pastenförmigen 2K-Zementen. Durch den erfindungsgemäßen zweikomponentigen Knochenzement ist es möglich, den Vermischungsvorgang der beiden Komponenten zeitlich vom Auspressvorgang zu trennen und bei Verwendung einer Kunststoffkartusche den Zementteig mit einer konventionellen, manuell angetriebenen Auspressvorrichtung, wie sie bisher bei Pulver-Flüssigkeits-Knochenzementen bekannt ist, z.B. der Palamix®-Zementierpistole (Heraeus Medical GmbH), auszupressen. Eine spezielle gasgetriebene Zementierpistole ist nicht erforderlich.

Die beiden Komponenten A und B stellen gesonderte Komponenten dar und können im nicht ausgehärteten Zustand separat gelagert werden. Die Komponente A stellt eine Paste dar und ist vorzugsweise bereits im Lagerzustand klebfrei. Die Komponente B stellt ein Pulver bzw. Pulvergemisch dar und kann daher einfach und preisgünstig hergestellt werden. Da der Röntgenopaker in der pulverförmigen Komponente B enthalten ist, können Probleme, die durch Sedimentation in Pasten auftreten, vermieden werden.

Der Ausdruck "umfassen", wie hier in der vorliegenden Erfindung verwendet, schließt auch "bestehen aus" und "im wesentlichen bestehen aus" mit ein. Unter Raumtemperatur (RT) wird eine Temperatur von 23°C verstanden.

Unter Partikelgröße D50 wird hier das Volumenmittel der Partikelgröße verstanden. D50 wird über ein Laserbeugungsverfahren mittels Streulichtbestimmung mit dem Laser Diffraction Particle Size Analyzer LS 13320 der Firma Beckman Coulter, USA, bestimmt.

Die zahlenmittlere Molmasse wird mittels Gelpermeationschromatographie (GPC) bestimmt.

In dem erfindungsgemäßen Knochenzement sind mindestens ein Hydroperoxid, mindestens ein tertiäres Amin, mindestens ein Schwermetallsalz und mindestens ein Reduktionsmittel verteilt auf die beiden Komponenten enthalten, wie nachstehend ausführlich erläutert. Diese vier Bestandteile bilden ein Redoxinitiatorsystem. Bei der Mischung der beiden Komponenten wird das Redoxinitiatorsystem aktiv und initiiert durch Radikalbildung die Polymerisation des Methylmethacrylats, wodurch der Knochenzement aushärtet.

Die Bestandteile des Redoxinitiatorsystems, d.h. Hydroperoxid, tertiäres Amin, Schwermetallsalz und Reduktionsmittel, sind alle in Methylmethacrylat bei Raumtemperatur löslich. Der Ausdruck "in Methylmethacrylat löslich" bedeutet hierbei, dass eine sichtklare Lösung gebildet wird. Dies wird visuell mit dem Auge bestimmt.

Bezüglich des in Methylmethacrylat unlöslichen, partikulären Polymers, das eine Partikelgröße D50 kleiner 50 µm, bedeutet "in Methylmethacrylat unlöslich", dass keine sichtklare Lösung gebildet wird. Dies wird visuell mit dem Auge bestimmt.

Die als Komponente A eingesetzte Paste umfasst Methylmethacrylat als Monomer. Methylmethacrylat wird auch als Methacrylsäuremethylester bezeichnet. Methylmethacrylat wird im Folgenden wie üblich auch als MMA abgekürzt. Der Gewichtsanteil von MMA in der Komponente A kann in breiten Bereichen variieren. Der Gewichtsanteil von MMA in der Komponente A liegt bevorzugt im Bereich von 30 bis 50 Gew.-%.

Die als Komponente A eingesetzte Paste umfasst ferner mindestens ein in Methylmethacrylat lösliches Polymer mit einer zahlenmittleren Molmasse kleiner 500.000 Dalton. Die zahlenmittlere Molmasse des mindestens einen in Methylmethacrylat löslichen Polymers beträgt bevorzugt mindestens 100.000 Dalton.

Es können prinzipiell alle in Methylmethacrylat löslichen Polymere verwendet werden. Es können ein oder mehrere in Methylmethacrylat lösliche Polymere verwendet werden. Das mindestens eine in Methylmethacrylat lösliche Polymer ist für die Erzielung der Klebfreiheit der pastenförmigen Komponente A wesentlich.

Beispiele für geeignete Polymere sind Polymethylmethacrylat und Copolymere von Methylmethacrylat mit einem oder mehreren damit copolymerisierbaren Monomeren, wie Methylacrylat, Styren und Ethylacrylat.

Das in Methylmethacrylat lösliche Polymer mit einer zahlenmittleren Molmasse kleiner 500.000 Dalton ist besonders bevorzugt ausgewählt aus Polymethylmethacrylat, Poly(methylmethacrylat-co-methylacrylat), Poly(methylmethacrylat-co-styren) und Mischungen davon.

Der Gewichtsanteil von dem mindestens einen in Methylmethacrylat löslichen Polymer mit einer zahlenmittleren Molmasse kleiner 500.000 Dalton in der Komponente A kann in breiten Bereichen variieren, wobei der Gewichtsanteil bevorzugt im Bereich von 12 bis 30 Gew.-% liegt.

Die als Komponente A eingesetzte Paste umfasst ferner mindestens ein in Methylmethacrylat unlösliches, partikuläres Polymer, das eine Partikelgröße D50 kleiner 50 µm hat. Es ist bevorzugt, dass das mindestens eine in Methylmethacrylat unlösliche, partikuläre Polymer eine Partikelgröße D50 von mindestens 0,5 µm aufweist. Das mindestens eine in Methylmethacrylat unlösliche, partikuläre Polymer weist bevorzugt eine Partikelgröße D50 im Bereich von 5 bis 10 µm auf.

Das in Methylmethacrylat unlösliche partikuläre Polymer weist vorzugsweise eine zum Methylmethacrylat nur geringfügig abweichende Dichte auf. Dadurch tritt eine Sedimentation der unlöslichen Polymerpartikel in der Paste A kaum bzw. praktisch nicht auf.

Als in Methylmethacrylat unlösliche Polymerteilchen mit einer Partikelgröße D50 kleiner 50 µm können prinzipiell alle in MMA unlösliche Polymere verwendet werden. Beispiele für geeignete Polymere sind vernetztes Polymethylmethacrylat, vernetztes Poly(methylmethacrylat-co-methacrylat) und vernetztes Poly(methylmethacrylat-co-styren).

Vernetztes Polymethylmethacrylat und vernetztes Poly(methylmethacrylat-co-methacrylat) stellen ein Copolymer von Methylmethacrylat oder von einer Mischung von Methylmethacrylat und Methacrylat mit einem oder mehreren damit copolymerisierbaren difunktionellen, trifunktionellen und/oder mehrfachfunktionellen Monomeren dar. Beispiele für die als Vernetzer fungierenden difunktionellen, trifunktionellen und mehrfachfunktionellen Monomeren sind Dimethacrylate, Trimethacrylate, Tetramethacrylate, Diacrylate, Triacrylate, Tetraacrylate, wobei Dimethacrylate bevorzugt sind.

Das in Methylmethacrylat unlösliche, partikuläre Polymer mit einer Partikelgröße D50 kleiner 50 µm ist bevorzugt vernetztes Polymethylmethacrylat. Besonders bevorzugt sind Copolymerisate aus Methylmethacrylat und Ethylenglykoldimethacrylat und Copolymerisate aus Methylmethacrylat und Propan-1,2-diol-dimethacrylat.

Der Gewichtsanteil von dem mindestens einen in Methylmethacrylat unlöslichen, partikulären Polymer mit einer Partikelgröße D50 kleiner 50 µm in der Komponente A kann in breiten Bereichen variieren, wobei der Gewichtsanteil bevorzugt im Bereich von 10 bis 40 Gew.-% liegt.

Die als Komponente A eingesetzte Paste umfasst ferner mindestens ein in Methylmethacrylat lösliches Hydroperoxid. Es können ein oder mehrere in Methylmethacrylat lösliche Hydroperoxide verwendet werden. Das Hydroperoxid dient als Radikalinitiator. Cumolhydroperoxid, t-Butyl-hydroperoxid oder Isoamylhydroperoxid und Mischungen davon sind als in Methylmethacrylat lösliches Hydroperoxid bevorzugt. Diese Hydroperoxide zeichnen sich durch eine hohe Temperatur- und Lagerstabilität aus.

Der Gewichtsanteil von dem mindestens einen in Methylmethacrylat löslichen Hydroperoxid in der Komponente A kann in breiten Bereichen variieren, wobei der Gewichtsanteil bevorzugt im Bereich von 0,01 bis 1,0 Gew.-% liegt.

Die als Komponente A eingesetzte Paste umfasst ferner mindestens ein in Methylmethacrylat lösliches tertiäres Amin. Es können ein oder mehrere in Methylmethacrylat lösliche tertiäre Amine verwendet werden. Bevorzugte Beispiele sind tertiäre aromatische Amine. Diese enthalten mindestens einen aromatischen Ring, insbesondere mindestens einen Benzolring. Das tertiäre Amin ist bevorzugt ein Toluidin und/oder ein Anilin.

Das tertiäre Amin ist besonders bevorzugt ausgewählt aus einem oder mehreren von N,N-Dimethyl-o-toluidin, N,N-Bis-hydroxyethyl-p-toluidin und N,N-Dimethylanilin.

Der Gewichtsanteil von dem mindestens einen in Methylmethacrylat löslichen tertiären Amin in der Komponente A kann in breiten Bereichen variieren, wobei der Gewichtsanteil bevorzugt im Bereich von 0,4 bis 4,0 Gew.-% liegt.

Die Kombination von tertiären Aminen und Hydroperoxiden in der pastenförmigen Komponente A ist möglich, weil für die Zersetzung des Hydroperoxids neben tertiären Aminen zusätzlich noch Schwermetallsalze und mindestens ein Reduktionsmittel erforderlich sind.

Im Rahmen der Erfindung können in der pastenförmigen Komponente A neben dem Methylmethacrylat gegebenenfalls ein oder mehrere andere monofunktionelle oder mehrfachfunktionelle Monomere enthalten sein, die mit Methylmethacrylat copolymerisierbar sind. Beispiele sind Alkylacrylate, Mono- und Dicarbonsäuren mit mindestens einer Olefingruppe, Dimethacrylate, Methacrylamid und 2-Hydroxyethylmethacrylat (HEMA). Bevorzugte Beispiele sind Ethylenglykoldimethacrylat, Propylenglykoldimethacrylat, Butandiol-1,4-dimethacrylat, Ethylmethacrylat, Hydroxyethylmethacrylat, Itaconsäuredimethylester und Methacrylamid. Sofern solche anderen Monomere eingesetzt werden, beträgt ihr Gewichtsanteil in der Komponente A bevorzugt nicht mehr als 5 Gew.-%.

Die Paste als Komponente A kann ferner gegebenenfalls weitere optionale Zusatzstoffe enthalten, z.B. eine oder mehrere farbgebende Substanzen und/oder einen oder mehrere pharmazeutische Wirkstoffe. Additive, wie farbgebende Substanzen und/oder pharmazeutische Wirkstoffe können in der pastenförmigen Komponente A und/oder der pulverförmigen Komponente B enthalten sein. Sofern farbgebende Substanzen und/oder pharmazeutische Wirkstoffe im erfindungsgemäßen Knochenzement enthalten sind, sind diese aber bevorzugt in der der pulverförmigen Komponente B enthalten.

Bei der farbgebenden Substanz handelt es sich bevorzugt um einen Lebensmittelfarbstoff oder einen Farblack. Beispiele sind E101, E104, E132, E141 (Chlorophyllin), E142, Riboflavin, Lissamingrün und Farblack Grün, das Aluminiumsalz einer Mischung aus E104 und E132.

Beispiele für geeignete pharmazeutische Wirkstoffe sind Antibiotika, Antiphlogistika, Steroide, Hormone, Wachstumsfaktoren, Bisphosphonate, Cytostatika und Genvektoren. Konkrete Beispiele sind nachstehend bei der Beschreibung der Komponente B genannt.

Die Paste als Komponente A kann einer Sterilisation unterworfen werden, z.B. mit einem Sterilisationsmittel. Die Paste kann z.B. mit dem in EP 2596812 A1 beschriebenen Sterilisationsverfahren durch Einwirkung von β-Propiolacton oder dessen Derivaten sterilisiert werden. Daneben ist auch eine Sterilisation durch Wasserstoffperoxid oder Wasserstoffperoxid-abspaltende Verbindungen, wie z.B. in DE 102009005534 B3 beschrieben, oder auch durch geeignete Persäuren möglich.

In einer bevorzugten Ausführungsform besteht die Paste als Komponente A im wesentlichen oder vollständig nur aus Methylmethacrylat, dem mindestens einen in Methylmethacrylat löslichen Polymer, dem mindestens einen in Methylmethacrylat unlöslichen partikulären Polymer, dem mindestens einen in Methylmethacrylat löslichen Hydroperoxid und dem mindestens einen in Methylmethacrylat löslichen tertiären Amin, wobei gegebenenfalls zusätzlich Sterilisationsmittel enthalten sind.

Der erfindungsgemäße Knochenzement enthält ferner ein Pulver bzw. Pulvergemisch als gesonderte Komponente B.

Die pulverförmige Komponente B umfasst mindestens einen partikulären Röntgenopaker mit einer Partikelgröße D50 kleiner 50 µm. Es können ein oder mehrere Röntgenopaker enthalten sein, die auch als Röntgenkontrastmittel bezeichnet werden. Röntgenopaker werden üblicherweise in Knochenzementen verwendet und sind im Handel erhältlich.

Bei dem partikulären Röntgenopaker mit einer Partikelgröße D50 kleiner 50 µm kann es sich z.B. um Metalloxide, z.B. Zirkoniumdioxid, Bariumsulfat, toxikologisch unbedenkliche Schwermetallpartikel, z.B. Tantal, Ferrit und Magnetit, oder toxikologisch unbedenkliche Calciumsalze, z.B. CaCO₃, CaSO₄ oder CaSO₄ 2H₂O, handeln, wobei Zirkoniumdioxid und/oder Bariumsulfat bevorzugt sind.

Der Gewichtsanteil von dem mindestens einen partikulären Röntgenopaker mit einer Partikelgröße D50 kleiner 50 µm in der Komponente B kann in breiten Bereichen variieren, wobei der Gewichtsanteil bevorzugt im Bereich von 50 bis 95 Gew.-% liegt.

Für die röntgenologische Darstellbarkeit des Polymethylmethacrylatzementes ist es notwendig, dass dieser mindestens einen röntgenopaken Stoff enthält. Röntgenopaker, wie Zirkoniumdioxid und Bariumsulfat, besitzen eine hohe Dichte.

Wenn Partikel mit hoher Dichte in Pasten enthalten sind, besteht die Gefahr einer Sedimentation dieser Partikel. Die Röntgenopaker befinden sich erfindungsgemäß in der pulverförmigen Komponente B. In der pastenförmigen Komponente A brauchen daher keine Röntgenopaker enthalten zu sein. Dadurch sind Probleme mit einer eventuellen Sedimentation der Röntgenoapker in der pastenförmigen Komponente A grundsätzlich ausgeschlossen. Weiterhin wirken die Röntgenopakerpartikel als Träger für das mindestens eine in Methylmethacrylat lösliche Schwermetallsalz und auch als Träger für das mindesten eine in Methylmethacrylat lösliche Reduktionsmittel, die ebenfalls in der pulverförmigen Komponente B enthalten sind.

Die pulverförmige Komponente B umfasst ferner mindestens ein in Methylmethacrylat lösliches Schwermetallsalz. Es können ein oder mehrere Schwermetallsalze eingesetzt werden. Das Schwermetallsalz dient der Radikalbildung. Es können alle üblichen in Methylmethacrylat löslichen Schwermetallsalze verwendet werden. Bei dem in Methylmethacrylat löslichen Schwermetallsalz handelt es sich insbesondere um eines, bei denen wenigstens zwei Oxidationszustände möglich sind. Schwermetallsalze schließen Schwermetallkomplexe ein. Schwermetalle haben bei 20°C in der Regel eine Dichte von mehr als 5 g/cm³.

Geeignete Schwermetallsalze sind z.B. Salze von Kupfer, Cobalt, Eisen oder Mangan, wobei Salze von Co(II) und Cu(II) besonders bevorzugt sind. Die Schwermetallsalze weisen als Anionen insbesondere organische Liganden oder organische Komplexliganden auf. Beispiele für geeignete Anionen sind Alkoholat, Acrylat, Methacrylat, Acetylacetonat und 2-Ethylhexanoat, wobei 2-Ethylhexanoat bevorzugt ist.

Bevorzugte Beispiele für in Methylmethacrylat lösliche Schwermetallsalze sind Kupfer(II)-2-ethylhexanoat, Kupfer(II)-methacrylat, Kupfer(II)-bisacetylacetonat, Cobalt(II)-2-ethylhexanoat und Cobalt(II)-bisacetylacetonat.

Der Gewichtsanteil von dem mindestens einen in Methylmethacrylat löslichen Schwermetallsalz in der Komponente B kann in breiten Bereichen variieren, wobei der Gewichtsanteil bevorzugt im Bereich von 0,5 bis 2,0 Gew.-% liegt.

Die pulverförmige Komponente B umfasst ferner mindestens in Methylmethacrylat lösliches Reduktionsmittel. Es können ein oder mehrere Reduktionsmittel eingesetzt werden. Es können alle hierfür üblichen Reduktionsmittel eingesetzt werden, wie z.B. in Methylmethacrylat lösliche Reduktone bzw. Endiole oder Imide.

Saccharin, Phthalimid, Succinimid, Maleinimid, Palmitoylascorbinsäure und Benzoin sind z.B. als in Methylmethacrylat lösliche Reduktionsmittel geeignet. Saccharin ist besonders bevorzugt, weil dieses kostengünstige Reduktionsmittel eine breite Verwendung als Süßstoff (E 954) findet und nach gegenwärtigem Kenntnisstand toxikologisch unbedenklich ist.

Der Gewichtsanteil von dem mindestens einen in Methylmethacrylat löslichen Reduktionsmittel in der Komponente B kann in breiten Bereichen variieren, wobei der Gewichtsanteil bevorzugt im Bereich von 2 bis 28 Gew.-% liegt.

In einer bevorzugten Ausführungsform enthält die pulverförmige Komponente B ferner Siliziumdioxid. Siliziumdioxid umfasst hier auch Mischoxide aus Siliziumdioxid und einem oder mehreren anderen Metalloxiden. Auch diese Mischoxide verfügen über oberflächliche Silanolgruppen, die zur Ausbildung von Wasserstoffbrückenbindungen befähigt sind. Der Gewichtsanteil an Siliziumdioxid in Komponente B kann in breiten Bereichen variieren und beträgt vorzugsweise 0,1 bis 20 Gew.-%. Das Siliziumdioxid weist vorzugsweise eine BET-Oberfläche von mindestens 40 m²/g, bevorzugter von 40 bis 450 m²/g, besonders bevorzugt von 40 bis 380 m²/g auf. Bevorzugt sind pyrogene Kieselsäuren, insbesondere hochdisperse, pyrogene Kieselsäuren, z.B. die Aerosile® von Evonik, Deutschland, z.B. AEROSIL® 380.

Durch den Einsatz von Siliziumdioxid kann die Paste A vorteilhafterweise niedrig viskos eingestellt werden kann und nach Vermischung mit der pulverförmigen Komponente B ein thixotroper Zementteig gebildet werden, der bei Scherung thixotropes Verhalten zeigt.

In der pulverförmigen Komponente B können ferner mit Vorteil ein oder mehrere optionale Zusatzstoffe enthalten sein, z.B. eine oder mehrere farbgebende Substanzen und/oder ein oder mehrere pharmazeutische Wirkstoffe, wie z.B. Antiinfektiva, Antiseptika, Antiphlogistika, Bisphosphonate, Wachstumsfaktoren, Antibiotika, Steroide, Hormone, Cytostatika und Genvektoren.

Bei der farbgebenden Substanz handelt es sich z.B. um Farbpigmente, Lebensmittelfarbstoffe oder Farblacke. Beispiele sind E101, E104, E132, E141 (Chlorophyllin), E142, Riboflavin, Lissamingrün und Farblack Grün, das Aluminiumsalz einer Mischung aus E104 und E132.

In dem trockenen Pulvergemisch der Komponente B lassen sich pharmazeutische Wirkstoffe, insbesondere Antiinfektiva, Antiseptika, Antiphlogistika, Bisphosphonate und Wachstumsfaktoren, relativ problemlos über einen längeren Zeitraum lagern. Als Antiinfektiva sind insbesondere Gentamicin, Tobramycin, Clindamycin, Vancomycin, Teicoplanin, Daptomycin und Fosfomycin bevorzugt, die in Form von in Wasser leicht löslichen Salzen oder auch in Form von in Wasser gering löslichen Salzen oder Komplexen eingesetzt werden können. Als Antiseptika kommen insbesondere Octenidindihydrochlorid, Polyhexanid, Calciumperoxid und Harnstoffperoxid in Frage.

Dadurch, dass die Zusatzstoffe im trockenen, ungelösten Zustand in der Komponente B enthalten sein können, werden Stabilitätsprobleme und damit Probleme der Lagerfähigkeit weitgehend vermieden im Vergleich zur Aufnahme in pastenförmige Komponenten von Knochenzementen, insbesondere solchen, die Monomere enthalten.

Die pulverförmige Komponente B kann in bekannter Weise durch Einwirkung von Gammastrahlung oder Ethylenoxid sterilisiert werden.

In einer bevorzugten Ausführungsform umfasst die pastenförmige Komponente A des zweikomponentigen Knochenzements, bezogen auf das Gesamtgewicht der Komponente A,
a1) 30 bis 50 Gew.-% Methylmethacrylat,
a2) 12 bis 30 Gew.-% mindestens eines in Methylmethacrylat löslichen Polymers mit einer zahlenmittleren Molmasse kleiner 500.000 Dalton,
a3) 10 bis 40 Gew.-% mindestens eines in Methylmethacrylat unlöslichen, partikulären Polymers, das eine Partikelgröße D50 kleiner 50 µm hat,
a4) 0,01 bis 1,0 Gew.-% mindestens eines in Methylmethacrylat löslichen Hydroperoxids und
a5) 0,4 bis 4,0 Gew.-% mindestens eines in Methylmethacrylat löslichen tertiären Amins.

In einer bevorzugten Ausführungsform umfasst die pulverförmige Komponente B des zweikomponentigen Knochenzements, bezogen auf das Gesamtgewicht der Komponente B,
b1) 50 bis 95 Gew.-% mindestens eines partikulären Röntgenopakers, der eine Partikelgröße kleiner 50 µm hat,
b2) 0,5 bis 2,0 Gew.-% mindestens eines in Methylmethacrylat löslichen Schwermetallsalzes und
b3) 2 bis 28 Gew.-% mindestens eines in Methylmethacrylat löslichen Reduktionsmittels,
sowie gegebenenfalls
b4) 2 bis 45 Gew.-% mindestens eines pharmazeutischen Wirkstoffs,
wobei, sofern in der Komponente B kein pharmazeutischer Wirkstoff enthalten ist, der Anteil der Komponente b1) vorzugsweise 70 bis 95 Gew.-% beträgt.

In einer besonders bevorzugten Ausführungsform sind in der pastenförmigen Komponente A und die pulverförmige Komponente B die Bestandteile jeweils in den vorstehend angegebenen Mengenanteilen vorhanden. Die Menge an Röntgenopaker in Komponente B kann, je nachdem, ob auch ein pharmazeutischer Wirkstoff enthalten ist, relativ stark variieren.

Die Erfindung betrifft auch ein Verfahren zur Herstellung eines gehärteten Zements mit dem erfindungsgemäßen zweikomponentigen Knochenzement, umfassend die Mischung der pastenförmigen Komponente A und der pulverförmigen Komponente B, um einen Zementteig zu bilden, der selbständig aushärtet. Die beiden Komponenten werden bevorzugt in einem Gewichtsverhältnis von 80 bis 90 Gewichtsteilen der Paste A zu 5 bis 10 Gewichtsteilen des Pulvers B vermischt.

Der Vermischungsvorgang der beiden Komponenten kann zeitlich vom Auspressvorgang getrennt werden. Die beiden Komponenten können z.B. in einer Kartusche, wie einer Kunststoffkartusche, vermischt werden, und der gebildete Zementteig mit einer Auspressvorrichtung ausgepresst werden. Hierfür eignen sich z.B. konventionelle, manuell angetriebene Auspressvorrichtungen, die auch bei konventionellen Pulver-Flüssigkeits-Knochenzementen eingesetzt werden, z.B. die Palamix®-Zementierpistole von Heraeus Medical GmbH.

Nach der Vermischung der Paste A mit der pulverförmigen Komponente B entsteht ohne Einhaltung einer Wartezeit sofort ein klebfreier, plastisch verformbarer Zementteig, der selbstständig durch radikalische Polymerisation aushärtet.

Der erfindungsgemäße Polymethylmethacrylat-Knochenzement bzw. der nach Vermischung der Komponenten A und B erhaltene Zementteig eignet sich z.B. insbesondere zur mechanischen Fixierung von Gelenkendoprothesen, zur Herstellung von temporären Spacern, zur Vertebroplastie, zur Kyphoplastie und zur Herstellung von Wirkstoffträgern zur lokalen Wirkstofffreisetzung.

Die Erfindung wird durch die nachstehenden Beispiele weiter erläutert, die die Erfindung jedoch in keiner Weise beschränken.

### Beispiele

### Herstelluna einer Paste A für die Beispiele 1-3

Es wurde für die Beispiele 1-3 jeweils eine Paste A mit folgender Zusammensetzung hergestellt:

| | |
|---|---|
| 36,25 g | Methylmethacrylat |
| 0,40 g | Methacrylamid |
| 0,30 g | 2-Hydroxyethylmethacrylat |
| 0,90 g | N,N-Dimethyl-p-toluidin |
| 2,10 g | Bis(2-hydroxyethyl)-p-toluidin |
| 0,10 g | 80%ige Cumolhydroperoxid-Lösung |
| 0,03 g | 2,6-Di-t-butyl-4-methyl-phenol |
| 19,40 g | Poly(methylmethacrylat-co-methylacrylat) Mn < 500.000 Dalton |
| 19,40 g | in MMA unlösliches, mit Ethylenglykoldimethacrylat vernetztes Polymethylmethacrylat mit einer Partikelgröße D50 kleiner 50 µm. |

Alle Substanzen wurden von der Firma Sigma-Aldrich bezogen, bis auf das lösliche Polymethylmethacrylat-co-methylacrylat und das unlösliche Polymethylmethacrylat. Die Komponenten wurden in einem Plastikbecher eingewogen, intensiv verrührt und nach Verschluss mit einem Schraubdeckel für mindestens 18 Stunden bei Raumtemperatur stehen gelassen.

### Herstellung einer pulverförmigen Komponente B für Beispiel 1

Zusammensetzung der pulverförmigen Komponente B:

| | |
|---|---|
| 4,80 g | Zirkoniumdioxid |
| 0,06 g | Kupfer(II)-2-ethylhexanoat |
| 0,05 g | Grünlack (Aluminiumfarblack von Indigodisulfonsäure und Chinolingelb) |
| 0,60 g | Saccharin |
| 1,18 g | Gentamicinsulfat |

Alle Substanzen der Komponente B wurden bis auf das Gentamicinsulfat von der Firma Sigma-Aldrich bezogen. Es wurde Gentamicinsulfat der Firma Fujian Fukang Ltd. (VR China) verwendet.

### Herstellug einer pulverförmigen Komponente B für Beispiel 2

Zusammensetzung der pulverförmigen Komponente B:

| | |
|---|---|
| 4,80 g | Zirkoniumdioxid |
| 0,06 g | Kupfer(II)-2-ethylhexanoat |
| 0,05 g | Grünlack (Aluminiumfarblack von Indigodisulfonsäure und Chinolingelb) |
| 0,60 g | Saccharin |
| 1,00 g | Vancomycinhydrochlorid |

Alle Substanzen der Komponente B wurden bis auf das Vancomycinhydrochlorid von der Firma Sigma-Aldrich bezogen. Das Vancomycinhydrochlorid stammt von der Firma Axellia Pharmaceuticals (Dänemark).

### Herstellung einer pulverförmigen Komponente für Beispiel 3

Zusammensetzung der pulverförmigen Komponente B:

| | |
|---|---|
| 4,80 g | Zirkoniumdioxid |
| 0,06 g | Kupfer(II)-2-ethylhexanoat |
| 0,05 g | Grünlack (Aluminiumfarblack von Indigodisulfonsäure und Chinolingelb) |
| 0,60 g | Saccharin |

### Beispiele 1 bis 3

### Vermischung und Applikation

Die Paste A für die Beispiele 1-3 wurde jeweils mit der pulverförmigen Komponente B der Beispiele 1 bis 3 intensiv miteinander verrührt, wobei jeweils die komplette Menge des Pulvers und der Paste wie vorstehend angegeben verwendet wurden. Es entstand bei allen Beispielen sofort ein grüner, klebfreier Zementteig. Dieser wurde in die Kartusche des Zementiersystems Palamix® überführt und durch Verwendung der manuell betriebenen Palamix®-Zementierpistole problemlos durch das übliche Austragsrohr des Zementiersystems daraus ausgepresst.

### Herstellung von Prüfkörpern

Die ISO 5833 fordert eine Biegefestigkeit von ≥ 50 MPa, ein Biegemodul von ≥ 1800 MPa und eine Biegefestigkeit von ≥ 70 MPa. Zur Prüfung der mechanischen Eigenschaften gemäß ISO 5833 der Pastenzemente der Beispiele 1-3 wurde Prüfkörper hergestellt. Die Paste A für die Beispiele 1-3 wurde jeweils mit der pulverförmigen Komponente B der Beispiele 1-3 intensiv miteinander verrührt, wobei jeweils die komplette Menge des Pulvers und der Paste wie vorstehend angegeben verwendet wurden. Es entstand bei allen Beispielen sofort eine grüner, klebfreier Zementteig, der nach wenigen Minuten exotherm aushärtete.

Es wurden mit dem Zementteig der Beispiele 1-3 jeweils streifenförmige Probekörper der Abmessung 75 mm x 10 mm x 3,3 mm für die Prüfung der Biegefestigkeit und des Biegemoduls gemäß ISO5833 hergestellt. Weiterhin wurden zylinderförmige Probekörper (Durchmesser 6 mm, Höhe 12 mm) für die Prüfung der Druckfestigkeit hergestellt.

Nach Lagerung der Probekörper bei 23°C, einer relativen Luftfeuchtigkeit von 50 %, über einen Zeitraum von 24 Stunden wurde die Biegefestigkeit, das Biegemodul und die Druckfestigkeit gemäß ISO 5833 bestimmt. Die Ergebnisse zeigen, dass die mechanischen Anforderungen der ISO5833 bezüglich der Biegefestigkeit, des Biegemoduls und der Druckfestigkeit von den Zementen der Beispiele 1-3 erfüllt wurden.

| Beispiel | Biegefestigkeit [MPa] | Biegemodul [MPa] | Druckfestigkeit [MPa] |
|---|---|---|---|
| 1 | 64,5 ± 2,4 | 2655 ± 59 | 100,4 ± 1,3 |
| 2 | 61,9 ± 2,4 | 2538 ± 97 | 99,8 ± 1,8 |
| 3 | 66,2 ± 1,0 | 2575 ± 67 | 96,5 ± 0,9 |

### Beispiele 4 bis 6

Die pastenförmige Komponente A wurde in gleicher Weise wie bei den Beispielen 1-3 hergestellt, wobei jedoch 0,1 Gew.-% Propiolacton zugesetzt wurde. Die pulverförmigen Komponenten B hatten die gleiche Zusammensetzung wie in den Beispielen 1-3. Die pastenförmige Komponente A wurde 3 Wochen nach ihrer Herstellung und Lagerung bei Raumtemperatur jeweils mit der pulverförmigen Komponente B der Beispiele 1-3 manuell vermischt, wobei jeweils die komplette Menge des Pulvers und der Paste wie vorstehend angegeben verwendet wurden, um jeweils den Zementteig der Beispiele 4-6 zu erhalten. Es entstand, vergleichbar wie bei den Beispielen 1-3, ein grüner, klebfreier Zementteig, der nach ungefähr 4 Minuten selbstständig exotherm aushärtete.

### Beispiel 7

**Zusammensetzung der Paste A:**

| | |
|---|---|
| 36,25 g | Methylmethacrylat |
| 0,40 g | Methacrylamid |
| 0,30 g | 2-Hydroxyethylmethacrylat |
| 0,90 g | N,N-Dimethyl-p-toluidin |
| 2,10 g | Bis(2-hydroxyethyl)-p-toluidin |
| 0,10 g | 80%ige Cumolhydroperoxid-Lösung |
| 0,03 g | 2,6-Di-t-butyl-4-methyl-phenol |
| 15,40 g | Poly(methylmetharylat-co-methylacrylat) Mn < 500.000 Dalton |
| 19,40 g | in MMA unlösliches, mit Ethylenglykoldimethacrylat vernetztes Polymethylmethacrylat mit einer Partikelgröße D50 kleiner 50 µm. |

**Zusammensetzung der Pulverförmigen Komponente B:**

| | |
|---|---|
| 4,80 g | Zirkoniumdioxid |
| 0,70 g | Aerosil® 380 (pyrogene Kieselsäure) |
| 0,06 g | Kupfer(II)-2-ethylhexanoat |
| 0,05 g | Grünlack (Aluminiumfarblack von Indigodisulfonsäure und Chinolingelb) |
| 0,60 g | Saccharin |
| 1,18 g | Gentamicinsulfat |

Die Paste A konnte problemlos manuell mit der Komponente B vermischt werden, wobei jeweils die komplette Menge des Pulvers und der Paste wie vorstehend angegeben verwendet wurden. Der Zementteig war klebfrei und konnte bei Scherung problemlos plastisch verformt werden. Der Zementteig ließ sich bis ca. 4 Minuten verarbeiten und härtete dann exotherm aus.

## Patentansprüche

1. Zweikomponentiger Knochenzement umfassend
A) eine Paste als Komponente A umfassend
a1) Methylmethacrylat,
a2) mindestens ein in Methylmethacrylat lösliches Polymer mit einer zahlenmittleren Molmasse kleiner 500.000 Dalton,
a3) mindestens ein in Methylmethacrylat unlösliches, partikuläres Polymer, das eine Partikelgröße D50 kleiner 50 µm hat,
a4) mindestens ein in Methylmethacrylat lösliches Hydroperoxid und
a5) mindestens ein in Methylmethacrylat lösliches tertiäres Amin
und
B) ein Pulver als Komponente B umfassend
b1) mindestens einen partikulären Röntgenopaker, der eine Partikelgröße D50 kleiner 50 µm hat,
b2) mindestens ein in Methylmethacrylat lösliches Schwermetallsalz und
b3) mindestens ein in Methylmethacrylat lösliches Reduktionsmittel.

2. Zweikomponentiger Knochenzement nach Anspruch 1, **dadurch gekennzeichnet, dass** die pastenförmige Komponente A
a1) 30 bis 50 Gew.-% Methylmethacrylat,
a2) 12 bis 30 Gew.-% mindestens eines in Methylmethacrylat löslichen Polymers mit einer zahlenmittleren Molmasse kleiner 500.000 Dalton,
a3) 10 bis 40 Gew.-% mindestens eines in Methylmethacrylat unlöslichen, partikulären Polymers, das eine Partikelgröße D50 kleiner 50 µm hat,
a4) 0,01 bis 1,0 Gew.-% mindestens eines in Methylmethacrylat löslichen Hydroperoxids und
a5) 0,4 bis 4,0 Gew.-% mindestens eines in Methylmethacrylat löslichen tertiären Amins
umfasst.

3. Zweikomponentiger Knochenzement nach Anspruch 1 oder Anspruch 2, **dadurch gekennzeichnet, dass** die pulverförmige Komponente B
b1) 50 bis 95 Gew.-% mindestens eines partikulären Röntgenopakers, der eine Partikelgröße kleiner 50 µm hat,
b2) 0,5 bis 2,0 Gew.-% mindestens eines in Methylmethacrylat löslichen Schwermetallsalzes und
b3) 2 bis 28 Gew.-% mindestens eines in Methylmethacrylat löslichen Reduktionsmittels
umfasst.

4. Zweikomponentiger Knochenzement nach einem der Ansprüche 1 bis 3, **dadurch gekennzeichnet, dass** die pulverförmige Komponente B ferner umfasst
b4) mindestens einen pharmazeutischen Wirkstoff, vorzugsweise 2 bis 45 Gew.-% mindestens eines pharmazeutischen Wirkstoffs.

5. Zweikomponentiger Knochenzement nach irgendeinem der Ansprüche 1 bis 4, **dadurch gekennzeichnet, dass** die pulverförmige Komponente B 0,1 bis 20 Gew.-% Siliziumdioxid enthält, das eine BET-Oberfläche von mindestens 40 m²/g, bevorzugt von 40 bis 450 m²/g, umfasst.

6. Zweikomponentiger Knochenzement nach irgendeinem der Ansprüche 1 bis 5, **dadurch gekennzeichnet, dass** das Mischungsverhältnis der beiden Komponenten 80 bis 90 Gewichtsteile der pastenförmigen Komponente A zu 5 bis 10 Gewichtsteilen der pulverförmigen Komponente B beträgt.

7. Zweikomponentiger Knochenzement nach irgendeinem der Ansprüche 1 bis 6, **dadurch gekennzeichnet, dass** das mindestens eine Hydroperoxid ausgewählt ist aus mindestens einem von Cumolhydroperoxid, tert.-Butylhydroperoxid und Isoamylhydroperoxid.

8. Zweikomponentiger Knochenzement nach irgendeinem der Ansprüche 1 bis 7, **dadurch gekennzeichnet, dass** das mindestens eine in Methylmethacrylat lösliche Polymer ausgewählt ist aus mindestens einem von Polymethylmethacrylat, Poly(methylmethacrylat-co-methylacrylat) und Poly(methylmethacrylat-co-styren).

9. Zweikomponentiger Knochenzement nach irgendeinem der Ansprüche 1 bis 8, **dadurch gekennzeichnet, dass** das in Methylmethacrylat unlösliche, partikuläre Polymer vernetztes Polymethylmethacrylat ist.

10. Zweikomponentiger Knochenzement nach irgendeinem der Ansprüche 1 bis 9, **dadurch gekennzeichnet, dass** das mindestens eine tertiäre Amin ausgewählt ist aus mindestens einem von N,N-Dimethyl-o-toluidin, N,N-bis-Hydroxyethyl-p-toluidin und N,N-Dimethylanilin.

11. Zweikomponentiger Knochenzement nach irgendeinem der Ansprüche 1 bis 10, **dadurch gekennzeichnet, dass** das mindestens eine Schwermetallsalz ausgewählt ist aus mindestens einem von Kupfer(II)-2-ethylhexanoat, Kupfer(II)-methacrylat, Kupfer(II)-bisacetylacetonat, Cobalt(II)-2-ethylhexanoat und Cobalt(II)-bisacetylacetonat.

12. Zweikomponentiger Knochenzement nach irgendeinem der Ansprüche 1 bis 11, **dadurch gekennzeichnet, dass** das mindestens eine Reduktionsmittel ausgewählt ist aus mindestens einem von Saccharin, Phthalimid, Succinimid, Maleinimid, Palmitoylascorbinsäure und Benzoin.

13. Zweikomponentiger Knochenzement nach irgendeinem der Ansprüche 1 bis 12, **dadurch gekennzeichnet, dass** der mindestens eine Röntgenopaker Zirkoniumdioxid und/oder Bariumsulfat ist.

14. Zweikomponentiger Knochenzement nach irgendeinem der Ansprüche 1 bis 13, **dadurch gekennzeichnet, dass** die pulverförmige Komponente B ferner mindestens einen Zusatzstoff ausgewählt aus farbgebenden Substanzen, wie Farbpigmenten oder Farblacken, und/oder pharmazeutischen Wirkstoffen, wie Antiinfektiva, Antiseptika, Antiphlogistika, Bisphosphonaten und Wachstumsfaktoren, umfasst.

15. Verfahren zur Herstellung eines gehärteten Zements mit einem zweikomponentigen Knochenzement nach irgendeinem der Ansprüche 1 bis 14, umfassend die Mischung der pastenförmigen Komponente A und der pulverförmigen Komponente B, um einen Zementteig zu erhalten, der selbstständig aushärtet, wobei bevorzugt 80 bis 90 Gewichtsteile der Paste A mit 5 bis 10 Gewichtsteilen des Pulvers B vermischt werden.

## Claims

1. A two-component bone cement, comprising
A) a paste as component A, comprising
a1) methyl methacrylate;
a2) at least one polymer that is soluble in methyl methacrylate and has a number average molar mass of less than 500,000 daltons;
a3) at least one particulate polymer that is insoluble in methyl methacrylate and has a particle size D50 of less than 50 µm;
a4) at least one hydroperoxide that is soluble in methyl methacrylate; and
a5) at least one tertiary amine that is soluble in methyl methacrylate;
and
B) a powder as component B, comprising
b1) at least one particulate radiodense substance that has a particle size D50 of less than 50 µm;
b2) at least one heavy-metal salt that is soluble in methyl methacrylate; and
b3) at least one reducing agent that is soluble in methyl methacrylate.

2. The two-component bone cement in accordance with Claim 1, **characterised in that** the pasty component A comprises
a1) 30 to 50 per cent by weight of methyl methacrylate;
a2) 12 to 30 per cent by weight of at least one polymer that is soluble in methyl methacrylate and has a number average molar mass of less than 500,000 daltons;
a3) 10 to 40 per cent by weight of at least one particulate polymer that is insoluble in methyl methacrylate and has a particle size D50 of less than 50 µm;
a4) 0.01 to 1.0 per cent by weight of at least one hydroperoxide that is soluble in methyl methacrylate; and
a5) 0.4 to 4.0 per cent by weight of at least one tertiary amine that is soluble in methyl methacrylate.

3. The two-component bone cement in accordance with Claim 1 or Claim 2, **characterised in that** the powdery component B comprises
b1) 50 to 95 per cent by weight of at least one particulate radiodense substance that has a particle size of less than 50 µm;
b2) 0.5 to 2.0 per cent by weight of at least one heavy-metal salt that is soluble in methyl methacrylate; and
b3) 2 to 28 per cent by weight of at least one reducing agent that is soluble in methyl methacrylate.

4. The two-component bone cement in accordance with any one of Claims 1 to 3, **characterised in that** the powdery component B further comprises
b4) at least one pharmaceutical active agent, preferably 2 to 45 per cent by weight of at least one pharmaceutical active agent.

5. The two-component bone cement in accordance with any one of Claims 1 to 4, **characterised in that** the powdery component B contains 0.1 to 20 per cent by weight of silicon dioxide that has a BET surface of at least 40 m²/g, preferably of 40 to 450 m²/g.

6. The two-component bone cement in accordance with any one of Claims 1 to 5, **characterised in that** the mixing ratio of the two components is 80 to 90 parts by weight of the pasty component A to 5 to 10 parts by weight of the powdery component B.

7. The two-component bone cement in accordance with any one of Claims 1 to 6, **characterised in that** the at least one hydroperoxide is selected from at least one of cumene hydroperoxide, tert-butyl hydroperoxide and isoamyl hydroperoxide.

8. The two-component bone cement in accordance with any one of Claims 1 to 7, **characterised in that** the at least one polymer that is soluble in methyl methacrylate is selected from at least one of polymethyl methacrylate, poly(methyl methacrylate-co-methyl acrylate) and poly(methyl methacrylate-co-styrene).

9. The two-component bone cement in accordance with any one of Claims 1 to 8, **characterised in that** the particulate polymer that is insoluble in methyl methacrylate is cross-linked polymethyl methacrylate.

10. The two-component bone cement in accordance with any one of Claims 1 to 9, **characterised in that** the at least one tertiary amine is selected from at least one of N,N-dimethyl-o-toluidine, N,N-bis-hydroxyethyl-p-toluidine and N,N-dimethylaniline.

11. The two-component bone cement in accordance with any one of Claims 1 to 10, **characterised in that** the at least one heavy-metal salt is selected from at least one of copper(II)-2-ethylhexanoate, copper(II)-methacrylate, copper(II)-bis-acetylacetonate, cobalt(II)-2-ethylhexanoate and cobalt(II)-bis-acetylacetonate.

12. The two-component bone cement in accordance with any one of Claims 1 to 11, **characterised in that** the at least one reducing agent is selected from at least one of saccharin, phthalimide, succinimide, maleinimide, ascorbic acid palmitate and benzoin.

13. The two-component bone cement in accordance with any one of Claims 1 to 12, **characterised in that** the at least one radiodense substance is zirconium dioxide and/or barium sulphate.

14. The two-component bone cement in accordance with any one of Claims 1 to 13, **characterised in that** the powdery component B further comprises at least one additive that is selected from colour-causing substances, such as coloured pigments or colour lakes, and/or from pharmaceutical active agents, such as anti-infectives, antiseptics, antiphlogistics, diphosphonates and growth factors.

15. A method for manufacturing a hardened cement with a two-component bone cement in accordance with any one of Claims 1 to 14, comprising the mixture of the pasty component A and the powdery component B, in order to obtain a cement dough which hardens by itself, wherein preferably 80 to 90 parts by weight of paste A are mixed with 5 to 10 parts by weight of powder B.

## Revendications

1. Ciment osseux bicomposant comprenant
A) une pâte comme composant A comprenant
a1) du méthylméthacrylate,
a2) au moins un polymère soluble dans du méthylméthacrylate avec une masse molaire moyenne en nombre inférieure à 500 000 daltons,
a3) au moins un polymère particulaire, insoluble dans du méthylméthacrylate qui a une taille particulaire D50 inférieure à 50 µm,
a4) au moins un hydroperoxyde soluble dans du méthylméthacrylate, et
a5) au moins une amine tertiaire soluble dans du méthylméthacrylate
et
B) une poudre comme composant B comprenant
b1) au moins un opacifiant radiographique particulaire qui a une taille particulaire D50 inférieure à 50 µm,
b2) au moins un sel de métal lourd soluble dans du méthylméthacrylate, et
b3) au moins un réducteur soluble dans du méthylméthacrylate.

2. Ciment osseux bicomposant selon la revendication 1, **caractérisé en ce que** le composant pâteux A comprend
a1) 30 à 50 % en poids de méthylméthacrylate,
a2) 12 à 30 % en poids d'au moins un polymère soluble dans du méthylméthacrylate avec une masse molaire moyenne en nombre inférieure à 500 000 daltons,
a3) 10 à 40 % en poids d'au moins un polymère particulaire, insoluble dans du méthylméthacrylate qui a une taille particulaire D50 inférieure à 50 µm,
a4) 0,01 à 1,0 % en poids d'au moins un hydroperoxyde soluble dans du méthylméthacrylate, et
a5) 0,4 à 4,0 % en poids d'au moins une amine tertiaire soluble dans du méthyl méthacrylate.

3. Ciment osseux bicomposant selon la revendication 1 ou la revendication 2, **caractérisé en ce que** le composant pulvérulent B comprend
b1) 50 à 95 % en poids d'au moins un opacifiant radiographique particulaire qui a une taille particulaire inférieure à 50 µm,
b2) 0,5 à 2,0 % en poids d'au moins un sel de métal lourd soluble dans du méthylméthacrylate, et
b3) 2 à 28 % en poids d'au moins un réducteur soluble dans du méthylméthacrylate.

4. Ciment osseux bicomposant selon une des revendications 1 à 3, **caractérisé en ce que** le composant pulvérulent B comprend en outre
b4) au moins une substance active pharmaceutique, de préférence 2 à 45 % en poids d'au moins une substance active pharmaceutique.

5. Ciment osseux bicomposant selon l'une quelconque des revendications 1 à 4, **caractérisé en ce que** le composant pulvérulent B contient 0,1 à 20 % en poids de dioxyde de silicium qui comprend une surface spécifique d'au moins 40 m²/g, de préférence de 40 à 450 m²/g.

6. Ciment osseux bicomposant selon l'une quelconque des revendications 1 à 5, **caractérisé en ce que** le rapport de mélange des deux composants se monte à 80 à 90 parties en poids du composant pâteux A sur 5 à 10 parties en poids du composant pulvérulent B.

7. Ciment osseux bicomposant selon l'une quelconque des revendications 1 à 6, **caractérisé en ce que** l'au moins un hydroperoxyde est sélectionné parmi au moins un élément parmi l'hydroperoxyde de cumène, le tert.-butylhydroperoxyde et l'isoamylhydroperoxyde.

8. Ciment osseux bicomposant selon l'une quelconque des revendications 1 à 7, **caractérisé en ce que** l'au moins un polymère soluble dans du méthylméthacrylate est sélectionné parmi au moins un élément parmi le polyméthylméthacrylate, le poly(méthylméthacrylate-co-méthylacrylate) et le poly(méthylméthacrylate-co-styrène).

9. Ciment osseux bicomposant selon l'une quelconque des revendications 1 à 8, **caractérisé en ce que** le polymère particulaire, insoluble dans du méthylméthacrylate est du polyméthylméthacrylate réticulé.

10. Ciment osseux bicomposant selon l'une quelconque des revendications 1 à 9, **caractérisé en ce que** l'au moins une amine tertiaire est sélectionnée parmi au moins un élément parmi la N,N-diméthyl-o-toluidine, la N,N-bis-hydroxyéthyl-p-toluidine et la N,N-diméthylaniline.

11. Ciment osseux bicomposant selon l'une quelconque des revendications 1 à 10, **caractérisé en ce que** l'au moins un sel de métal lourd est sélectionné parmi au moins un élément parmi le 2-éthylhexanoate de cuivre(II), le méthacrylate de cuivre(II), le bisacétylacétonate de cuivre(II), le 2-éthylhexanoate de cobalt(II) et le bisacétylacétonate de cobalt(II).

12. Ciment osseux bicomposant selon l'une quelconque des revendications 1 à 11, **caractérisé en ce que** l'au moins un réducteur est sélectionné parmi au moins un élément parmi la saccharine, le phtalimide, le succinimide, le maléinimide, l'acide palmitoylascorbique et la benzoïne.

13. Ciment osseux bicomposant selon l'une quelconque des revendications 1 à 12, **caractérisé en ce que** l'au moins un opacifiant radiographique est le dioxyde de zirconium et/ou le sulfate de baryum.

14. Ciment osseux bicomposant selon l'une quelconque des revendications 1 à 13, **caractérisé en ce que** le composant pulvérulent B comprend en outre au moins un additif sélectionné parmi des substances colorantes, comme des pigments colorés ou des laques colorées, et/ou des substances actives pharmaceutiques, comme des anti-infectieux, antiseptiques, anti-inflammatoires, biphosphonates et facteurs de croissance.

15. Procédé de fabrication d'un ciment durci avec un ciment osseux bicomposant selon l'une quelconque des revendications 1 à 14, comprenant le mélange du composant pâteux A et du composant pulvérulent B pour obtenir une pâte de ciment qui durcit de manière autonome, dans lequel 80 à 90 parties en poids de la pâte A sont de préférence mélangées à 5 à 10 parties en poids de la poudre B.
